# EUROPÄISCHE PATENTSCHRIFT

(11) **EP 2 190 349 B1**
(45) Veröffentlichungstag und Bekanntmachung des Hinweises auf die Patenterteilung: **11.11.2015**
(21) Anmeldenummer: 08802003.7
(22) Anmeldetag: 11.09.2008
(51) Int. Cl.: A61B 5/03, A61B 5/00

(54) **SENSORSYSTEM ZUR MESSUNG, ÜBERTRAGUNG VERARBEITUNG UND DARSTELLUNG EINES HIRNPARAMETERS**
SENSOR SYSTEM FOR MEASURING, TRANSFERRING, PROCESSING AND DISPLAYING A BRAIN PARAMETER
SYSTÈME CAPTEUR POUR MESURER, TRANSMETTRE, TRAITER ET REPRÉSENTER UN PARAMÈTRE CÉRÉBRAL

(30) Priorität: 28.09.2007 DE 102007046694
(43) Veröffentlichungstag der Anmeldung: 02.06.2010
(60) Teilanmeldung: 13150166.0
(73) Patentinhaber: RAUMEDIC AG, 95213 Münchberg (DE)
(72) Erfinder: TAUBER, Karsten, 65550 Limburg (DE); VON FALKENHAUSEN, Christian, 53340 Meckenheim (DE); KUNZE, Gerd, 08297 Zwönitz (DE); GÖHLER, Karl-Heinz, 08297 Zwönitz (DE)
(74) Vertreter: Hofmann, Matthias
(86) Internationale Anmeldenummer: PCT/EP2008/007442
(87) Internationale Veröffentlichungsnummer: WO 2009/043431

(56) Entgegenhaltungen:
- EP-A1- 1 922 997
- DE-U1-202007 002 592
- US-A- 3 998 213
- US-A- 4 676 255
- US-A- 5 873 840
- US-A1- 2004 054 271
- US-A1- 2005 090 756
- US-A1- 2005 107 716
- US-A1- 2005 137 652
- US-B1- 6 468 219
- US-B1- 7 191 013

## Beschreibung

Die Erfindung betrifft ein Sensorsystem zur Messung, Übertragung, Verarbeitung und Darstellung eines Hirnparameters nach dem Oberbegriff des Anspruchs 1.

Ferner betrifft die Erfindung eine Kopfhaube für ein derartiges Sensorsystem.

Ein Sensorsystem der eingangs genannten Art ist bekannt aus der WO2004/023993 A1. Weitere Sensorsysteme sind bekannt aus der EP 1 312 302 A2, der DE 696 34 810 T2, der DE 197 05 474 A1, der DE 696 34 689 T2, der US 4,519,401 A, der US 2003/0023146 A1, *der* US 5 873 840*, der* US 2005/0090756 A1*, der* US 4 676 255 *und der* US 2005/0137652 A1*.*

Aus der DE 102 15 115 A1 ist eine EEG-Kappe mit Sensoren bekannt, die zur Ausbildung einer Messeinrichtung in einer Vorrichtung zur reaktiven automatischen nicht invasiven gesteuerten bzw. geregelten elektromagnetischen Prävention epileptischer Anfälle in vivo ein Sensorgitter bilden.

Hirnparametermessungen finden insbesondere mit dem Ziel statt, die Notwendigkeit für spätere Therapiemaßnahmen abzuklären. Ein Anwendungsbeispiel ist hierfür eine Hirndruck-Langzeitmessung zur Klärung der Frage, ob ein Hydrocephaluspatient ein Shuntventil benötigt und wie ein derartiges Ventil hinsichtlich seiner Dimensionierung ausgelegt werden muss. Hierfür ist eine Datenerfassung des Hirndruckverlaufes über einen längeren Zeitraum, beispielsweise über Nacht oder über mehrere Tage erforderlich, um aus der Dynamik des Hirndrucks (ICP, Intracranial Pressure) eine entsprechende Diagnose ableiten zu können.

Der Einsatz der bisher bekannten Sensorsysteme zur Bearbeitung derartiger Fragestellungen ist für den Patienten sehr unbequem und beschwerlich.

Es ist eine Aufgabe der vorliegenden Erfindung, ein Sensorsystem der eingangs genannten Art derart weiterzubilden, dass dessen Einsatz auch über einen längeren Messzeitraum für den Patienten komfortabel bleibt.

Diese Aufgabe wird erfindungsgemäß gelöst durch ein Sensorsystem mit den im Anspruch 1 angegebenen Merkmalen.

Erfindungsgemäß wurde erkannt, dass diejenigen Komponenten, die zum Empfangen der Messdaten vom implantierten Hirnparametersensor erforderlich sind, so leicht und bauklein gestaltet werden können, dass sie ohne weiteres an einer Kopfhaube befestigt werden können. Die Kopfhaube schützt die Empfangseinheit. Der Patient kann sich mit der Kopfhaube im Wesentlichen frei bewegen, muss also während der Messdauer nicht stationär untergebracht sein. Die Kopfhaube stellt eine exakte Relativposition der Empfangseinheit zur Sendeeinheit sicher.

*Eine Gitternetz-Kopfhaube hat sich beim Einsatz im Zusammenhang mit EEG-(Elektroenzephalographie-)Systemen bewährt. Eine entsprechende Gitternetz-Kopfhaube kommt auch, nach Ausrüstung mit einer entsprechenden Empfangseinheit, beim Sensorsystem zum Einsatz.*

Eine Antenne nach Anspruch 2 ist besonders leicht und bauklein und damit komfortabel zu tragen.

Eine Klettverbindung nach Anspruch 3 erlaubt eine ortsflexible Relativpositionierung zur Justierung der Empfangseinheit relativ zur Sendeeinheit. Hierzu kann die Empfangseinheit gegenüber der Kopfhaube stufenlos verlagert werden, bis eine gewünschte Soll-Signalstärke bei der Übertragung zwischen der Sendeeinheit und der Empfangseinheit erreicht ist. Insbesondere ist es möglich, Symmetrieachsen der Sendeeinheit und der Empfangseinheit miteinander praktisch vollständig zur Überdeckung zu bringen, was eine sehr gute Qualität der Signalübertragung gewährleistet.

Ein Gehäusering nach Anspruch 4 gewährleistet eine sichere Anlage der Empfangseinheit am Patientenkopf. Da die Sendeeinheit in der Regel zwischen der Kopfhaut und der Kalotte untergebracht ist, stellt die Sendeeinheit eine deutlich fühlbare Erhebung am Patientenkopf dar. Der Gehäusering ist bevorzugt so dimensioniert, dass er diese Erhebung zumindest abschnittsweise umschließt, was die Relativpositionierung der Sendeeinheit zur Empfangseinheit verbessert.

In dem am Patientenkopf anlegbaren Gehäuse bzw. Gehäusering kann nur ein Teil der die Empfangseinheit aufbauenden Komponenten untergebracht sein. Bevorzugt ist lediglich die Antenne der Empfangseinheit im Gehäuse bzw. Gehäusering untergebracht, wohingegen die anderen Komponenten der Empfangseinheit beispielsweise über eine kabelgebundene oder auch über eine kabellose Signalverbindung mit der Antenne in Verbindung stehen. Die am Patientenkopf anlegbare Komponente kann dann leicht und kompakt ausgestaltet sein.

Ausgestaltungen nach den Ansprüchen *5 und 6* verbessern die Anlage der Empfangseinheit an der Sendeeinheit und damit die Relativpositionierung dieser beiden Einheiten zueinander.

Eine Integration nach Anspruch 7 vermeidet insbesondere, dass der Patient neben der Kopfhaube noch zusätzliche Komponenten beispielsweise am Gürtel tragen muss.

Die Vorteile einer Kopfhaube nach Anspruch 8 entsprechen denen, die vorstehend unter Bezugnahme auf das erfindungsgemäße Sensorsystem bereits ausgeführt wurden.

Ein Ausführungsbeispiel der Erfindung wird nachfolgend anhand der Zeichnung näher erläutert. In dieser zeigen:
- Fig. 1: ein Sensorsystem zur Messung, Übertragung, Verarbeitung und Darstellung eines Hirnparameters;
- Fig. 2: schematisch einen Schnitt durch eine auf einen Patientenkopf aufgesetzte Kopfhaube des Sensorsystems im Bereich einer an der Kopfhaube festgelegten Antenne.

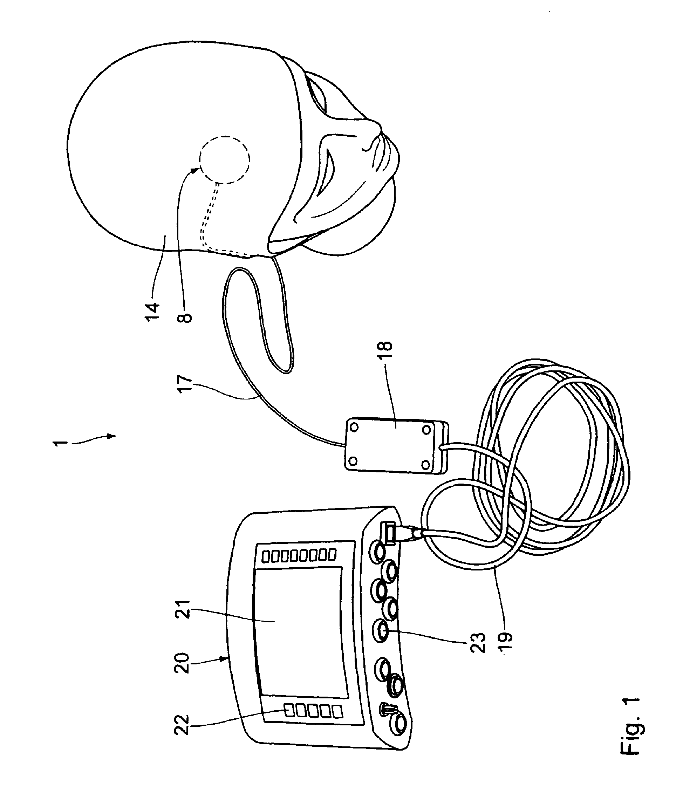

Ein in der Fig. 1 insgesamt dargestelltes Sensorsystem 1 dient zur Messung, Übertragung, Verarbeitung und Darstellung eines Hirnparameters, insbesondere eines Hirndrucks.

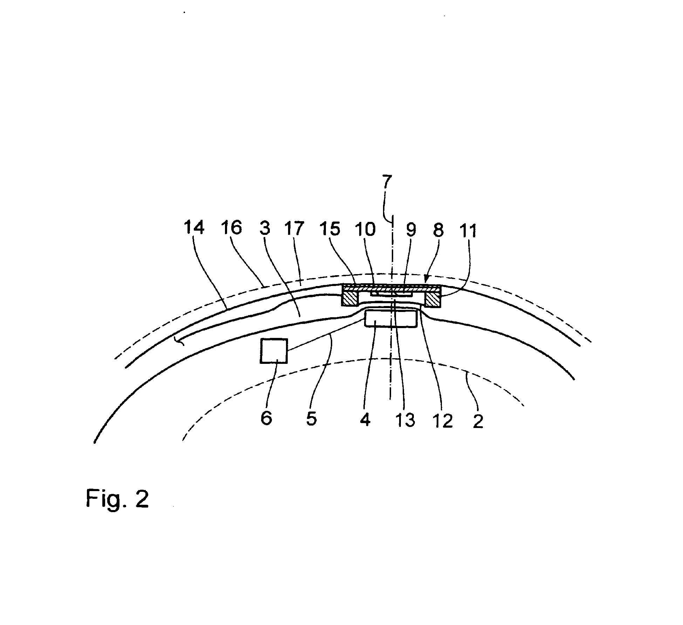

Zum Sensorsystem 1 gehört ein nicht näher dargestellter, in einen Patientenkopf implantierbarer Hirnparametersensor. Der ausschnittsweise Querschnitt der Fig. 2 zeigt Komponenten des implantierten Himparametersensors, die zwischen einer gestrichelt in der Fig. 2 angedeuteten Kalotte 2 und der Kopfhaut 3 eines Patienten angeordnet sind. Hierzu gehört ein drahtloser Sensortransponder 4 mit einer Antennenspule. Der Sensortransponder 4 wird nachfolgend auch als Sendeeinheit bezeichnet. Die Sendeeinheit 4 steht über eine Leitung 5 sowie eine nicht dargestellte Gleichrichtung mit einem Kondensator 6 in elektrischem Kontakt steht. Die Darstellung nach Fig. 2 ist äußerst schematisch und nicht maßstabsgerecht.

Der Sensortransponder 4 hat keine eigene Energiequelle. Der Sensortransponder 4 weist zusätzlich einen nicht näher dargestellten A/D-Wandler auf. Dieser dient zur Digitalisierung der vom Sensortransponder 4 weiterzugebenden Sensor-Messdaten.

Eine Rotationssymmetrieachse 7 der Antennenspule der Sendeeinheit 4 ist in der Fig. 2 gestrichelt angedeutet. Die Achse 7 verläuft bedingt durch die Anordnung der Sendeeinheit 4 im Wesentlichen normal zur Außenwand der Kalotte 2 im Bereich der Auflage der Antennenspule der Sendeeinheit 4 auf dieser.

Über eine nicht dargestellte Signalverbindung ist die Sendeeinheit 4 mit einem Sensorelement des Hirnparametersensors verbunden. Das Sensorelement ist beispielsweise als durch die Kalotte 2 hindurch geführter Katheter mit einem Druckmesskopf ausgeführt.

Der Sendeeinheit 4 zugeordnet ist eine äußere Sende/Empfangseinheit 8 des Sensorsystems 1, die nachfolgend auch nur als Empfangseinheit oder als Reader bezeichnet wird. Die Empfangseinheit 8 ist außerhalb des Patientenkopfes angeordnet. Die Empfangseinheit 8 hat eine Antenne 9, die drahtlos zur Energieversorgung sowie zur Datenübermittlung mit der Sendeeinheit 4 in Signalverbindung steht. Die Antenne 9 ist als Teil einer gedruckten Schaltung (PCB, printed circuit board) auf einer dünnen Platine 10 mit einer Stärke zwischen 0,8 mm und 1,5 mm aufgedruckt. Auch eine noch dünnere Platine ist möglich. Zur Verbesserung der Anpassungsfähigkeit der Empfangseinheit 8 kann die Platine 10 als flexible, also als biegsame Platine ausgebildet sein. Die Antenne 9 hat ebenfalls die Form einer Spule, deren Rotationssymmetrieachse mit der Achse 7 zusammenfällt. Die Spulenwindungen der Einheiten 4, 8 können insbesondere spiralförmig angeordnet sein.

Die Platine 10 ist an einem ringförmigen Gehäuse 11 mit einem Durchmesser von einigen cm befestigt. Auch die Rotationssymmetrieachse des Gehäuses 11 fällt mit der Achse 7 zusammen. Der Befestigungsort der Platine 10 ist auf einer von der Kopfhaut 3 abgewandten Seite des Gehäuses 11.

Ferner ist am Gehäuse 11 an der der Kopfhaut 3 zugewandten Seite eine flexible Membran 12 festgelegt. Die Membran 12 liegt somit zwischen der Antenne 9 und der Kopfhaut 3.

Zwischen der Antenne 9 und der Membran 12 ist eine Schicht 13 aus einem nachgiebigen, formveränderlichen Material, insbesondere aus einem fließfähigen und hochviskosem Material, insbesondere aus einem Gel angeordnet.

Beim Auflegen der Empfangseinheit 8 über das Gehäuse 11 auf die Kopfhaut 3 im Bereich der Sendeeinheit 4 passt sich aufgrund einer gezielten Verdrängung der Schicht 13 die Membran 12 an die Form des Patientenkopfes an dieser Stelle an, so dass ein korrekter Sitz der Empfangseinheit 8 relativ zur Sendeeinheit 4 gewährleistet ist.

Die Empfangseinheit 8 ist an der Innenseite einer Kopfhaube 14 festgelegt, die ebenfalls Teil des Sensorsystems 1 ist. Zur Befestigung der Empfangseinheit 8 an der Kopfhaube 14 dient eine Klettverbindung mit einem Klettband 15, das auf der der Kopfhaube 14 zugewandten Seite auf das Gehäuse 11 und die Platine 10 der Empfangseinheit 8 aufgeklebt ist. Über diese Klettverbindung lässt sich die Empfangseinheit 8 an einer beliebigen Stelle auf der Innenseite der Kopfhaube 14 anbringen, so dass eine gewünschte Relativpositionierung der Empfangseinheit 8 zur Sendeeinheit 4 möglich ist. Die Kopfhaube 14 ist aus einem dehnbaren und eng am Patientenkopf anliegenden Gaze oder Baumwollmaterial. Die Kopfhaube 14 ist im Schnitt an die Form des Patientenkopfes angepasst. Die Kopfhaube 14 kann zur Fixierung am Patientenkopf ein in der Zeichnung nicht dargestelltes Kinnband aufweisen. Zusätzlich zur Kopfhaube 14 kann auch noch eine in Fig. 2 gestrichelt angedeutete äußere Überhaube 16 vorgesehen sein, die Kräfte von außen aufnimmt und somit die dann innere Kopfhaube 14 gegen ein unerwünschtes Verrutschen relativ zur Kalotte 2 sichert.

Über ein Signalkabel 17, welches abschnittsweise zwischen der Kopfhaut 3 und der Kopfhaube 14 verläuft, steht die Antenne 9 der Empfangseinheit 8 mit einem Datenlesemodul 18 in Signalverbindung. Das Datenlesemodul 18 umfasst eine Steuereinheit zur Steuerung des implantierten Himparametersensors über die Empfangseinheit 8, über die eine bidirektionale Datenübertragung möglich ist. Ferner hat das Datenlesemodul 18 eine Hochfrequenzquelle zur Erzeugung einer Trägerfrequenz von 13,56 MHz. Das Datenlesemodul 18 hat zudem einen Signalspeicher. Das Datenlesemodul 18 hat in etwa die Größe einer Zigarettenschachtel und kann vom Patienten am Gürtel getragen werden.

Über ein weiteres Signalkabel 19 steht das Datenlesemoduls 18 mit einer Datenverarbeitungs- und -darstellungseinrichtung 20 in Signalverbindung. Letztere hat ein Display 21 sowie eine Mehrzahl von Bedienknöpfen 22 und Anschlussbuchsen 23, insbesondere mindestens eine USB-Schnittstelle. Die Datenverarbeitungs- und -darstellungseinrichtung 20 ist mit einer externen Spannungsquelle verbindbar. Alternativ oder zusätzlich kann die Datenverarbeitungs- und -darstellungseinrichtung 20 eine interne Energiequelle, beispielsweise in Form einer Batterie oder eines aufladbaren Akkumulators, aufweisen. Über diese Energiequelle werden das Datenlesemodul 18, die Empfangseinheit 8 und drahtlos während eines Messvorgangs auch die Sendeeinheit 4 versorgt.

Bei der Signalverbindung zwischen der Antenne 9 und dem Datenlesemodul 18 einerseits und dem Datenlesemodul 18 und der Datenverarbeitungs- und -darstellungseinrichtung 20 andererseits kann es sich um eine USB-Schnittstelle oder auch um eine RS232-Schnittstelle, also um ein digitales Interface, handeln.

Ein Hirnparameter-Messvorgang, der über einen längeren Zeitraum, beispielsweise im Verlauf mehrerer Tage, regelmäßig wiederholt wird, läuft folgendermaßen ab:

Das Datenlesemodul 18 sendet über die Empfangseinheit 8 zunächst ein HF-Signal an die Sendeeinheit 4. Das HF-Signal wird gleichgerichtet und lädt den Kondensator 6 zur zeitweiligen Energieversorgung der Sendeeinheit 4 auf. Nach diesem Ladevorgang wird HF-Signal deaktiviert. Anschließend nimmt der Hirnparametersensor, versorgt über den Kondensator 6, das Hirnparameter-Messsignal, beispielsweise einen Hirndruckwert, auf. Anschließend wird das HF-Signal wieder aktiviert und die Sendeeinheit 4 sendet mit dem HF-Signal als Trägerfrequenz den über den A/D-Wandler digitalisierten Messwert als digital moduliertes Signal an die Empfangseinheit 8. In der Empfangseinheit 8 erfolgt eine Überführung bzw. Dekodierung des modulierten HF-Signals in ein digitales, PC-kompatibles Signal. Je nach dem Schnittstellen-Standard, der beim Sensorsystem 1 eingesetzt wird, kann es sich beispielsweise um ein USB- oder um ein RS232-Signal handeln. Dieses überführte Messsignal wird an das Datenlesemodul 18 über das Signalkabel 17 übertragen. Das übertragene Signal wird dann über das Signalkabel 19 an die Datenverarbeitungs- und -darstellungseinrichtung 20 zur weiteren Verarbeitung und Darstellung übermittelt.

An Stelle einer Gaze- oder Baumwoll-Ausführung der Kopfhaube 14 und gegebenenfalls der Überhaube 16 kann die Kopfhaube 14 auch als Netz aus einer Mehrzahl von Einzelbändern oder -schläuchen ausgebildet sein. Durch diese Einzelbänder oder -schläuche wird dann ein Gitternetz vorgegeben. Die Empfangseinheit 8 kann dann Befestigungsmittel an den Einzelbändern oder -schläuchen aufweisen, die es insbesondere erlauben, die Empfangseinheit 8 an Kreuzungspunkten dieses Gitternetzes zu befestigen. Entsprechende Ausführungen von Kopfhauben aus Einzelbändern oder -schläuchen sind im Zusammenhang mit EEG-Kopfhauben bekannt.

Bei einer weiteren Variante des Sensorsystems 1 sind die Empfangseinheit 8 und das Datenlesemodul 18 als integrierte Baueinheit ausgeführt und an der Kopfhaube 14 befestigt. Zur Gewichtsverteilung dieser integrierten Baueinheit kann das Datenlesemodul auch von der Empfangseinheit 8 separat innen an der Kopfhaube 14 befestigt sein, beispielsweise an einer der Empfangseinheit 8 gegenüberliegenden Position. Die Empfangseinheit 8 und das Dateniesemodul 18 können dann über ein kurzes Signalkabel miteinander verbunden sein.

Eine entsprechende Integration der Empfangseinheit 8 ist auch mit Einzelkomponenten des Datenlesemoduls 18 möglich, beispielsweise mit der HF-Quelle oder mit der Steuereinheit des Datenlesemoduls 18. Die restlichen Komponenten sind dann wiederum über eine Signalverbindung mit einem externen, also außerhalb der Kopfhaube 14 angebrachten, Modul verbunden.

Bei diesen integrierten Varianten, bei denen die Empfangseinheit 8 noch zusätzliche Komponenten des Datenlesemoduls 18 aufweist, ist es auch möglich, dass die Empfangseinheit 8 eine eigene Energieversorgung, beispielsweise in Form einer Knopfzelle aufweist. In diesem Fall kann auf eine kabelgebundene Signalverbindung auch verzichtet werden und an Stele des Signalkabels 17 kann eine drahtlose Signalverbindung, beispielsweise eine Bluetooth-Signalverbindung zum Einsatz kommen.

Bei einer weiteren Variante des Sensorsystems 1 hat das Datenlesemodul 18 eine eigene Energieversorgung und an Stelle einer kabelgebundenen Signalverbindung mit der Datenverarbeitungs- und -darstellungseinrichtung 20 (Signalkabel 19) liegt eine drahtlose Signalverbindung vor, die beispielsweise ebenfalls über eine Bluetooth-Verbindung realisiert sein kann.

Die Datenverarbeitungs- und -darstellungseinrichtung 20 muss nicht ständig mit dem Datenlesemodul 18 in Signalverbindung stehen. Es reicht aus, wenn die Datenverarbeitungs- und -darstellungseinrichtung 20 die aufgenommenen Messdaten aus dem Datenlesemodul 18 nach Abschluss der beispielsweise mehrtätigen Messzeit ausliest. Erst dann ist eine entsprechende Datenverbindung erforderlich, so dass der Patient während der Messzeit nur die Kopfhaube 14 mit der Empfangseinheit 8 und das Datenlesemodul 18 tragen muss.

Die ortsflexible Positionierung der Empfangseinheit 8 über die Klettverbindung mit dem Klettband 15 ermöglicht eine exakte Ausrichtung der Empfangseinheit 8 zur Sendeeinheit 4, so dass die Spulenachsen dieser beiden Einheiten 4, 8 zusammenfallen. Dies gewährleistet eine optimale Signalübertragung zwischen den Einheiten 4 und 8.

Bei einer nicht näher dargestellten Ausführung ist die Empfangseinheit 8 mit einem ringförmigen Gehäuse 11 ausgeführt, in das eine ebenfalls ringförmige Antenne 9 eingearbeitet ist. Diese Ringantenne wird bei dieser Ausführungsform am Kopf des Patienten aufgesetzt. Die restliche Empfangseinheit, also die weiteren Komponenten, die bei der Ausführung nach den Figuren 1 und 2 insbesondere auf der gedruckten Schaltung auf der Platine 10 ausgeführt sind, sind mit der Ringantenne über ein Datenkabel verbunden. Diese restliche Empfangseinheit kann beispielsweise am Gürtel getragen oder auf einem Labortisch aufgestellt werden. Diese Trennung der Ringantenne von der sonstigen Empfangseinheit verringert die Baugröße und das Gewicht der am Kopf des Patienten aufzusetzenden Komponente.

Das Gehäuse der Ringantenne dieser nicht weiter dargestellten Ausführung kann charakteristische Ausbuchtungen und/oder Mulden und/oder sonstige Vertiefungen aufweisen, die beim manuellen Aufsetzen der Ringantenne zur Erleichterung der Positionierung von dieser dienen. Es können beispielsweise drei Ausbuchtungen und/oder Mulden und/öder Vertiefungen vorliegen, die in etwa gleich verteilt am Außenumfang der Ringantenne angeordnet sind.

Das Gehäuse der Ringantenne kann radial verlaufende Vertiefungen an der vom Patientenkopf in der aufgesetzten Stellung abgewandten Oberseite der Ringantenne aufweisen. Diese radial verlaufenden Vertiefungen können als Befestigungshilfen für ein Klettband zur Fixierung der Ringantenne an der Kopfhaube 14 dienen.

## Patentansprüche

1. Sensorsystem (1) zur Messung, Übertragung, Verarbeitung und Darstellung eines Hirnparameters
- mit mindestens einem implantierbaren Hirnparametersensor mit einer drahtlosen Sendeeinheit (4) zur Messung des Himparameters,
- mit mindestens einer Empfangseinheit (8) mit einer Antenne (9), die mit der Sendeeinheit (4) in drahtloser Signalverbindung steht,
- mit mindestens einem Datenlesemodul (18), welches mit der Antenne (9) über eine Signalverbindung (17) in Signalverbindung steht,
- mit mindestens einer Datenverarbeitungs- und -darstellungseinrichtung (20), welche mit dem Datenlesemodul (18) in Signalverbindung (19) steht,
**gekennzeichnet durch** eine Kopfhaube (14), an der die Empfangseinheit (8) zur Vorgabe einer Relativpositionierung relativ zur Sendeeinheit (4) festgelegt ist, wobei die Kopfhaube (14) eine Mehrzahl von Einzelbändern oder -schläuchen aufweist, die ein Gitternetz vorgeben, wobei die Empfangseinheit (8) an Kreuzungspunkten des Gitternetzes festlegbar ist.

2. Sensorsystem (1) nach Anspruch 1, **dadurch gekennzeichnet, dass** die Antenne (9) Teil einer gedruckten Schaltung ist.

3. Sensorsystem (1) nach Anspruch 1 oder 2, **dadurch gekennzeichnet, dass** die Empfangseinheit (8) an der Kopfhaube (14) über eine Klettverbindung (15) festgelegt ist.

4. Sensorsystem (1) nach einem der Ansprüche 1 bis 3, **dadurch gekennzeichnet, dass** die Empfangseinheit (8) über einen Gehäusering (11) an einem Patientenkopf anlegbar ist.

5. Sensorsystem (1) nach einem der Ansprüche 1 bis 4, **gekennzeichnet durch** eine flexible Membran (12), über die die Empfangseinheit (8) an dem Patientenkopf anlegbar ist.

6. Sensorsystem (1) nach Anspruch 5, **dadurch gekennzeichnet, dass** zwischen der flexiblen Membran (12) und der Antenne (9) eine formveränderliche Schicht (13), insbesondere eine Gelschicht, angeordnet ist.

7. Sensorsystem (1) nach einem der Ansprüche 1 bis 6, **dadurch gekennzeichnet, dass** das Datenlesemodul (18) und die Empfangseinheit (8) als integrierte Baueinheit ausgeführt und an der Kopfhaube (14) festlegbar sind.

8. Kopfhaube (14) mit einer Empfangseinheit (8) zum Einsatz in einem Sensorsystem (1) nach einem der Ansprüche 1 bis 7.

## Claims

1. Sensor system (1) for measuring, transmitting, processing and displaying a brain parameter
- with at least one implantable brain parameter sensor with a wireless transmission unit (4) for measuring the brain parameter,
- with at least one receiving unit (8) with an antenna (9), which is in wireless signal connection with the transmission unit (4),
- with at least one data read module (18), which is in signal connection with the antenna (9) by means of a signal connection (17),
- with at least one data processing and display device (20), which is in signal connection (19) with the data read module (18),
**characterised by** a head cap or a head hood (14), on which the receiving unit (8) is fixed to predetermine a relative positioning relative to the transmission unit (4), wherein the head cap or the head hood (14) has a plurality of individual bands or individual tubes, which predetermine a grid network, wherein the receiving unit (8) is fixable on crossing points of the grid network.

2. Sensor system (1) according to claim 1, **characterised in that** the antenna (9) is part of a printed circuit.

3. Sensor system (1) according to claim 1 or 2, **characterised in that** the receiving unit (8) is fixed to the head cap or the head hood (14) by a hook-and-loop connection (15).

4. Sensor system (1) according to any one of claims 1 to 3, **characterised in that** the receiving unit (8) is placeable by means of a housing ring (11) on a patient's head.

5. Sensor system (1) according to any one of claims 1 to 4, **characterised by** a flexible membrane (12), by means of which the receiving unit (8) is placeable on the patient's head.

6. Sensor system (1) according to claim 5, **characterised in that** a layer (13) of variable shape, in particular a gel layer, is arranged between the flexible membrane (12) and the antenna (9).

7. Sensor system (1) according to any one of claims 1 to 6, **characterised in that** the data read module (18) and the receiving unit (8) are configured as an integral modular unit and are fixable to the head cap or the head hood (14).

8. Head cap or head hood (14) with a receiving unit (8) for use in a sensor system (1) according to any one of claims 1 to 7.

## Revendications

1. Système de capteur (1) pour la mesure, la transmission, le traitement et la représentation d'un paramètre cérébral
- avec au moins un capteur de paramètre cérébral pouvant être implanté, doté d'une unité émettrice (4) sans fil pour la mesure du paramètre cérébral,
- avec au moins une unité réceptrice (8) dotée d'une antenne (9) qui est en communication par signal sans fil avec l'unité émettrice (4),
- avec au moins un module de lecture de données (18), lequel est en communication par signal avec l'antenne (9) par l'intermédiaire d'une liaison par signal (17),
- avec au moins une installation de traitement et de représentation de données (20) qui est en communication par signal (19) avec le module de lecture de données (18),
**caractérisé par** un couvre-chef (14) sur lequel est fixée l'unité réceptrice (8) pour la prescription d'un positionnement relatif par rapport à l'unité émettrice (4), où le couvre-chef (14) présente une multiplicité de bandes ou de tubulures individuelles, qui définissent un réseau sous forme de grille, où l'unité réceptrice (8) peut être implantée au niveau de points d'intersection du réseau en forme de grille.

2. Système de capteur (1) selon la revendication 1, **caractérisé en ce que** l'antenne (9) est une partie d'un circuit imprimé.

3. Système de capteur (1) selon les revendications 1 ou 2, **caractérisé en ce que** l'unité réceptrice (8) est fixée sur le couvre-chef (14) par l'intermédiaire d'une fixation par velcro (15).

4. Système de capteur (1) selon l'une des revendications 1 à 3, **caractérisé en ce que** l'unité réceptrice (8) peut être accolée à la tête du patient par l'intermédiaire d'un anneau de boîtier (11).

5. Système de capteur (1) selon l'une des revendications 1 à 4, **caractérisé par** une membrane flexible (12) par l'intermédiaire de laquelle l'unité réceptrice (8) peut être accolée à la tête du patient.

6. Système de capteur (1) selon la revendication 5, **caractérisé en ce qu'**une couche (13) pouvant changer de forme, notamment, une couche de gel, est disposée entre la membrane flexible (12) et l'antenne (9).

7. Système de capteur (1) selon l'une des revendications 1 à 6, **caractérisé en ce que** le module de lecture de données (18) et l'unité réceptrice (8) sont conçus sous la forme d'un composant intégré et peuvent être fixés sur le couvre-chef (14).

8. Couvre-chef (14) avec une unité réceptrice (8) pour une utilisation dans un système de capteur (1) selon l'une des revendications 1 à 7.
